# EUROPEAN PATENT APPLICATION

(11) **EP 4 570 822 A1**
(43) Date of publication of application: **18.06.2025**
(21) Application number: 23461695.1
(22) Date of filing: 15.12.2023
(51) Int. Cl.: C07K 16/12, A61P 31/04, C07K 16/02, A61K 39/395

(54) **IMMUNIZING AND/OR MEDICAL PREPARATION FOR USE IN PREVENTING AND/OR TREATING INFLAMMATORY CONDITIONS OF COW UDDER**

(71) Applicant: Zaklad Badawczo-Wdrozeniowy Osrodka Salmonella "Immunolab" Sp. z o.o., 80-822 Gdansk (PL)
(72) Inventor: LIEDER, Dorota, 83-050 Babi Dó (PL); G O NICKI, Krzysztof, 83-050 Babi Dó (PL)
(74) Representative: JD&P Patent Attorneys Joanna Dargiewicz & Partners

(57) **Abstract**

The subject matter of the invention is an immunizing and/or therapeutic preparation for use in the prevention and/or treatment of udder inflammation (mastitis) in cows, which contains IgY antibodies isolated from chicken egg yolks, directed against *Escherichia coli, Klebsiella pneumoniae, Klebsiella oxytoca*, *Staphylococcus aureus, Streptococcus uberis* and *Streptococcus dysgalactiae.* The subject matter of the invention is also a method for obtaining the above preparation.

## Description

The present invention relates to an immunizing and/or therapeutic preparation for use in the prevention and/or treatment of udder inflammations (mastitis) in cows.

Mastitis, udder inflammation, is a chronic, latent or acute inflammation of the mammary gland in animals; term also sometimes used in medicine for breast inflammation. A functional disorder of the mammary gland, proceeding under the influence of microorganisms that have entered the body. Clinical form (acute and chronic), subclinical (septic and aseptic). Udder inflammation causes a variety of qualitative changes in the milk, primarily reducing its technological suitability.

Each year, about 30% of Poland's dairy cattle population shows clinical mastitis symptoms and about 40% of cows show subclinical symptoms. Globally, this problem affects 20-45% of dairy cows, generating economic losses of €200/cow. According to the Central Statistical Office of Poland, the number of dairy cows in June 2019 was 2,233,000, which is about 1/3 of the cattle population in Poland. The methods of therapy and immunoprophylaxis of mastitis developed so far are not effective enough.

Mastitis is a multifactorial disease, both the host (cow), the pathogens and the environment are important. Pathogens most commonly isolated from cows include: *Staphylococcus aureus, Streptococcus agalactiae, Streptococcus uberis, Trueperella pyogenes, Escherichia coli.* During the course of the disease, the main changes in the milk (reducing its technological suitability) are: increased somatic cell count, concentration of chloride ions, level of the LDH enzyme, as well as the presence of secretory changes in the form of clots. Mastitis causes a decrease in a cow's milk yield by up to 17%. In addition to economic losses, all cases of the clinical form of mastitis cause pain and stress to the animal. The most important risk factors for mastitis incidence identified in the EFSA (2018) report are poor bedding hygiene and poorly designed, used or maintained milking equipment. Currently, treatment for clinical forms of the disease includes the administration of antibiotics and anti-inflammatory drugs. Such therapies show variable efficacy, due to increasing bacterial resistance to antibiotics, as well as untargeted treatment. Mastitis is a disease caused by several different pathogens, and therefore finding an effective antibiotic is problematic. In addition, there is an increase in drug resistance in dairy cattle, primarily to *S*. *aureus.* Too frequent administration of antibiotics can result in immune disorders, cause allergies and skin lesions, exacerbate or over-inhibit the inflammatory action of cytokines. The frequent use of antibiotics in the rearing of livestock from which food products are obtained is one of the sanitary and hygienic risks affecting public health. Preparations immunizing against mastitis are also available on the market, but they are designed for administration by injection.

European patent application EP3184120 relates to a therapy for treating or preventing several diseases in animals, based on the administration of a highly concentrated avian derived immunoglobulin formulation, obtained from the egg yolk from hens previously hyper-immunized with a vaccine formulation comprising infectious agents or toxin antigens, a light mineral oil and a particulate adjuvant.

European patent application EP1485414 relates to a multifunctional complex directed to the induction of a specific immunostimulatory response, preferably phagocytosis, against at least one target pathogenic agent. The complex according to the invention contains at least one target-recognizing component comprising a molecule that specifically binds to the desired target agent, an immunoactive component comprising an immunostimulatory agent; and optionally a linking component that links the targeting component and the immunoactive component. The complex according to the invention provides effective therapeutic prevention and treatment of various pathological conditions, such as mastitis in cows and furunculosis in fish. The invention further relates to compositions containing the targeting complex, methods of treatment and their applications.

Therefore, there is still a need in the field for new therapeutic and immunizing preparations that are not antibiotics.

So, the objective of the present invention was to develop new preparations for the prophylaxis and treatment of udder inflammation (mastitis) in cows, which could be an alternative to conventional chemotherapeutics and antibiotics.

This objective was achieved by the subject matter of the present invention, which is an immunizing and/or therapeutic preparation for use in the prevention and/or treatment of udder inflammation (mastitis) in cows, characterized in that it contains IgY antibodies isolated from chicken egg yolks, directed against:
a) *Escherichia coli*
b) *Klebsiella pneumoniae* and *Klebsiella oxytoca*
c) *Staphylococcus aureus*
d) *Streptococcus uberis*
e) *Streptococcus dysgalactiae*

Preferably, the IgY antibodies are combined in a weight ratio of 1:1:1:1:1.

Preferably, the IgY antibodies are isolated from chicken eggs yolks from hens immunized with the strain:
a) *Escherichia coli* having the *stx1* and/or eae and *stx1* and/or ipaH and/or *elt* genes,
b) *Klebsiella pneumoniae* serotype K1 having the gene encoding aerobactin and Klebsiella oxytoca having the *uge* and *kfu* genes,
c) *Staphylococcus aureus* expressing the surface proteins IsdC, EsxA, IsdA, IsdB, Bap, SdrD, SrdC,
d) *Streptococcus uberis* having *sua, pauA*/*skc, gapC, hasA, hasB, hasC, oppF* and/or *cfu* and *gapC* genes,
e) *Streptococcus dysgalactiae* having eno and napr genes.

Preferably, the preparation is for intramammary administration.

Preferably, the preparation contains a carrier in the form of a hydrogel

Preferably, the hydrogel contains 10% glycerol in water, 1% hydroxyethylcellulose powder with a viscosity of 1500 to 2500 cps.

Preferably, the preparation contains saline and thimerosal 0.01% as additives.

Preferably, the final protein concentration in the preparation is 1 to 20 mg/mL for each antigen, preferably 2 to 10 mg/mL, more preferably 4 to 8 mg/mL.

Preferably, the preparation is administered according to the following scheme:

| Administration | Age of the animal | Composition of the intramammary dose |
|---|---|---|
| 2x daily for 6 days | cows older than 24 months | 20 mg/mL of specific IgY antibodies (mixed in 1:1:1:1:1 weight ratio of 4 mg/mL of each antibody type) in a 1% hydrogel |
| 2x daily for 3 days | cows older than 24 months | 40 mg/mL of specific IgY antibodies (mixed in 1:1:1:1:1 weight ratio of 8 mg/mL of each antibody type) in a 0.5% hydrogel |

The subject matter of the present invention is also a method for obtaining the preparation defined above comprising the steps in which:
- A hydrogel containing 10% glycerol in water is prepared, heated to at least 95 °C, then after cooling to about 80-90 °C while stirring, hydroxyethylcellulose with a viscosity of 1500 to 2500 cps is added in powder form to obtain a concentration of 0.25 to 2%, preferably 0.5 to 1%, and the hydrogel is stirred until completely cooled;
- The IgY mixture and sterile saline are added to the resulting hydrogel until the final IgY concentration in the preparation is 1 to 20 mg/mL for each antigen, preferably 2 to 10 mg/mL, more preferably 4 to 8 mg/mL.

The results of tests confirming the properties of the developed therapeutic preparation are shown in the drawings, where:
Fig. 1 shows the results of testing the pathogen growth inhibition by IgY;
Fig. 2 shows the results of testing the pathogen growth inhibition by IgY;
Fig. 3 shows the results of testing the purity of IgY antibodies isolated from chicken egg yolks;
Figs. 4a-4d show the results of testing the neutralization of *E. coli* bacteria by IgY antibodies encapsulated in hydrogel;
Figs. 5a-5 show the results of neutralizing *S. dysgalactiae* bacteria with IgY antibodies encapsulated in hydrogel;

The authors of the present invention have developed immunological preparations for the prevention and treatment of udder inflammations (mastitis) in cows, which can be an alternative to conventional chemotherapeutics and antibiotics. A preparation against bacterial udder inflammation (mastitis) in cows has been developed, which is administered in hydrogel form as an intramammary infusion.

The preparation according to the present invention contains IgY antibodies directed against the most common mastitis pathogens:
a) *Escherichia coli*
b) *Klebsiella pneumoniae* and *Klebsiella oxytoca*
c) *Staphylococcus aureus*
d) *Streptococcus uberis*
e) *Streptococcus dysgalactiae*

**Target animal species:** The product is intended for cows of all ages, including heifers.

### Therapeutic indication for individual target animal species:

Preparation. Passive immunization of healthy cows and heifers to reduce the incidence of clinical mammary gland inflammation caused by the most common mastitis pathogens: *Escherichia coli, Klebsiella pneumoniae, Klebsiella oxytoca, Staphylococcus aureus, Streptococcus uberis, Streptococcus dysgalactiae,* as well as to reduce the somatic cell count in quarters infected with the above pathogens and to reduce losses in milk production caused by infection of the udder.

Laboratory and field studies have demonstrated the protective and therapeutic effects of the product, which inactivates pathogens present in the cow udder and mobilizes the cow immune system to fight the developing infection.

### DETAILED DESCRIPTION OF THE INVENTION

The veterinary therapeutic preparation according to the present invention contains as active ingredients IgY antibodies directed against the mastitis pathogens *Escherichia coli, Klebsiella ssp., Staphylococcus aureus, Streptococcus uberis, Streptococcus dysgalactiae* combined in a weight ratio of 1:1:1:1:1.

Route of administration: intramammary.

Additional substances: saline, thimerosal 0.01%, hydrogel (10% glycerol in water, 1% hydroxyethylcellulose powder with medium viscosity, molecular weight of 170,000, viscosity of 1500-2500 cps).

**Table 1. Preparation administration scheme**

| Administration | Age of the animal | Composition of the intramammary dose |
|---|---|---|
| 2x daily for 6 days | cows older than 24 months | 20 mg/mL of specific IgY antibodies (mixed in 1:1:1:1:1 weight ratio of 4 mg/mL of each antibody type) in a 1% hydrogel |
| 2x daily for 3 days | cows older than 24 months | 40 mg/mL of specific IgY antibodies (mixed in 1:1:1:1:1 weight ratio of 8 mg/mL of each antibody type) in a 0.5% hydrogel |

| | | |
|---|---|---|
| IgY antibodies directed against mastitis pathogens were extracted from egg yolks from hens immunized with the following strains: | | |

### 1. Escherichia coli

### Immunolab Collection Number: 4M01

Origin of the strain: Strain isolated from milk from a cow with mastitis symptoms. The sample was collected in March 2021. Received from PAS Olsztyn.

Storage and transportation conditions: The milk sample was collected into a sterile container and frozen. After collecting more samples, they were transported under refrigeration to Immunolab company.
Growth media: Soybean casein broth, Columbia Agar
Differentiation media: MacConkey Agar
Culture media: LB (similar growth also in enriched and soybean casein broths)
Bacterial growth: Growth abundant, large white-gray colonies, in liquid medium after inactivation easy to pellet by centrifugation
Biochemical features:

| **Name of the method:** ENTERO test 24N | | |
|---|---|---|
| **Identified strain:** *Escherichia coli* | | |
| **Evaluation:** Excellent identification (ID: 99.90, T-index: 0.782) | | |
| **Test** | **Abbreviation** | **Reaction** |
| Urease | URE | - |
| Arginine | ARG | - |
| Ornithine | ORN | + |
| Lysine | LYS | + |
| Hydrogen sulfide | H₂S | - |
| Simmons' citrate | SCI | - |
| Malonate | MAL | - |
| β-Galactosidase | ONP | + |
| Salicin | SAL | - |
| Sorbitol | SOR | - |
| Melibiose | MLB | + |
| Cellobiose | CEL | - |
| Lactose | LAC | + |
| Trehalose | TRE | + |
| Mannitol | MAN | + |
| β-Glucuronidase | GLR | + |
| Dulcitol | DUL | - |
| Adonitol | ADO | - |
| Arabitol | ART | - |
| Sucrose | SUC | + |
| Inositol | INO | - |
| Raffinose | RAF | + |
| Esculin | ESL | - |
| β-Xylosidase | bXY | - |

Antibiotic resistance:

| **Antibiotic abbreviation and concentration** | **Name of the antibiotic** |
|---|---|
| AMC 30 | Amoxicillin plus clavulanic acid |
| CL 30 | Cephalexin |
| CEP 30 | Cefoperazone |
| TE 30 | Tetracycline |
| N 30 | Neomycin |
| S 300 | Streptomycin |
| K 30 | Kanamycin |
| CN 10 | Gentamicin |
| MAR 5 | Marbocyl/Marbofloxacin |
| ENR 5 | Enrofloxacin |

| **Strain tested** | **Antibiotic** | | | | | | | | | |
|---|---|---|---|---|---|---|---|---|---|---|
| | **AMC 30** | **CL 30** | **CEP 30** | **TE 30** | **N 30** | **S 300** | **K 30** | **CN 10** | **MAR 5** | **ENR 5** |
| **4 MO1 E. *coli*** | S | MS | MS | MS | R | MS | R | MS | S | MS |

| | | | | | | | | | | |
|---|---|---|---|---|---|---|---|---|---|---|
| S: sensitive MS: medium sensitive R: resistant | | | | | | | | | | |

### Genetic features:

The strain has the *stx1* gene, which is responsible for the production of Shiga toxin in shigatoxigenic E coli (STEC) strains.

### 2. Escherichia coli

### Immunolab Collection Number: 13 MO₂

Origin of the strain: Strain isolated from milk from a cow with mastitis symptoms. The sample was collected in March 2021, designated 11/2021 MO, 187. Received from PAS Olsztyn.

Storage and transportation conditions: The milk sample was collected into a sterile container and frozen. After collecting more samples, they were transported under refrigeration to Immunolab company.
Growth media: Soybean casein broth, Columbia Agar
Differentiation media: Chromogenic Coliform Agar
Culture media: LB (similar growth also in enriched and soybean casein broths)
Bacterial growth: Growth abundant, large white-gray colonies, in liquid medium after inactivation easy to pellet by centrifugation
Biochemical features:

| **Name of the method:** ENTERO test 24N | | |
|---|---|---|
| **Identified strain:** *Escherichia coli* | | |
| **Evaluation:** Excellent identification (ID: 100, T-index: 0.926) | | |
| **Test** | **Abbreviation** | **Reaction** |
| Urease | URE | - |
| Arginine | ARG | - |
| Ornithine | ORN | + |
| Lysine | LYS | + |
| Hydrogen sulfide | H₂S | - |
| Simmons' citrate | SCI | - |
| Malonate | MAL | - |
| β-Galactosidase | ONP | + |
| Salicin | SAL | - |
| Sorbitol | SOR | + |
| Melibiose | MLB | + |
| Cellobiose | CEL | - |
| Lactose | LAC | + |
| Trehalose | TRE | + |
| Mannitol | MAN | + |
| β-Glucuronidase | GLR | + |
| Dulcitol | DUL | - |
| Adonitol | ADO | - |
| Arabitol | ART | - |
| Sucrose | SUC | + |
| Inositol | INO | - |
| Raffinose | RAF | + |
| Esculin | ESL | - |
| β-Xylosidase | bXY | - |

Antibiotic resistance:

| **Antibiotic abbreviation and concentration** | **Name of the antibiotic** |
|---|---|
| AMC 30 | Amoxicillin plus clavulanic acid |
| CL 30 | Cephalexin |
| CEP 30 | Cefoperazone |
| TE 30 | Tetracycline |
| N 30 | Neomycin |
| S 300 | Streptomycin |
| K 30 | Kanamycin |
| CN 10 | Gentamicin |
| MAR 5 | Marbocyl/Marbofloxacin |
| ENR 5 | Enrofloxacin |

| **Strain tested** | **Antibiotic** | | | | | | | | | |
|---|---|---|---|---|---|---|---|---|---|---|
| | **AMC 30** | **CL 30** | **CEP 30** | **TE 30** | **N 30** | **S 300** | **K 30** | **CN 10** | **MAR 5** | **ENR 5** |
| **13 R *E. coli*** | MS | S | R | S | MS | S | MS | MS | MS | MS |

| | | | | | | | | | | |
|---|---|---|---|---|---|---|---|---|---|---|
| S: sensitive MS: medium sensitive R: resistant | | | | | | | | | | |

### Genetic features:

The strain has the eae and *stx1* genes. The eae gene encodes the outer membrane protein intimin, which is responsible for adhesion to the host intestinal epithelium in enteropathogenic *E coli* (EPEC) strains, while the *stx1* gene, encodes Shiga toxin in shigatoxigenic *E coli* (STEC) strains.

### 3. Escherichia coli

### Immunolab Collection Number: 16L₁

Origin of the strain: Strain isolated from milk from a cow with mastitis symptoms. The sample was collected in February 2021. The sample was collected by and received from vet. Przemys 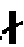 aw Warmowski from Pawe 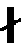 Somionek's farm near Kartuz.

Storage and transportation conditions: The milk sample was collected into a sterile container and transported under refrigeration to Immunolab company.
Growth media: Soybean casein broth, Columbia Agar
Differentiation media: MacConkey Agar
Culture media: LB (similar growth also in enriched and soybean casein broths)
Bacterial growth: Growth abundant, large white-gray colonies, in liquid medium after inactivation easy to pellet by centrifugation
Biochemical features:

| **Name of the method:** ENTERO test 24N | | |
|---|---|---|
| **Identified strain:** *Escherichia coli* | | |
| **Evaluation:** Excellent identification (ID: 100, T-index: 0.954) | | |
| **Test** | **Abbreviation** | **Reaction** |
| Urease | URE | - |
| Arginine | ARG | - |
| Ornithine | ORN | + |
| Lysine | LYS | + |
| Hydrogen sulfide | H₂S | - |
| Simmons' citrate | SCI | - |
| Malonate | MAL | - |
| β-Galactosidase | ONP | + |
| Salicin | SAL | - |
| Sorbitol | SOR | + |
| Melibiose | MLB | + |
| Cellobiose | CEL | - |
| Lactose | LAC | + |
| Trehalose | TRE | + |
| Mannitol | MAN | + |
| β-Glucuronidase | GLR | + |
| Dulcitol | DUL | - |
| Adonitol | ADO | - |
| Arabitol | ART | - |
| Sucrose | SUC | - |
| Inositol | INO | - |
| Raffinose | RAF | + |
| Esculin | ESL | - |
| P-Xylosidase | bXY | - |

Antibiotic resistance:

| **Antibiotic abbreviation and concentration** | **Name of the antibiotic** |
|---|---|
| AMC 30 | Amoxicillin plus clavulanic acid |
| CL 30 | Cephalexin |
| CEP 30 | Cefoperazone |
| TE 30 | Tetracycline |
| N 30 | Neomycin |
| S 300 | Streptomycin |
| K 30 | Kanamycin |
| CN 10 | Gentamicin |
| MAR 5 | Marbocyl/Marbofloxacin |
| ENR 5 | Enrofloxacin |

| **Strain tested** | **Antibiotic** | | | | | | | | | |
|---|---|---|---|---|---|---|---|---|---|---|
| | **AMC 30** | **CL 30** | **CEP 30** | **TE 30** | **N 30** | **S 300** | **K 30** | **CN 10** | **MAR 5** | **ENR 5** |
| **16 L1 *E*. *coli*** | S | MS | S | R | R | MS | R | MS | R | R |

| | | | | | | | | | | |
|---|---|---|---|---|---|---|---|---|---|---|
| S: sensitive MS: medium sensitive R: resistant | | | | | | | | | | |

### Genetic features:

The strain has the *ipaH* gene, typical of enteroinvasive *E coli* (EIEC) strains.

### 4. Escherichia coli

### Immunolab Collection Number: PAN 4/1

Origin of the strain: A strain isolated from milk from a cow with mastitis symptoms. The sample was collected in May 2020 from a farm in the Warmian-Masurian voivodeship. The sample was obtained from PAS Olsztyn.

Storage and transportation conditions: The milk sample was collected into a sterile container and frozen. After collecting more samples, they were transported under refrigeration to Immunolab company.
Growth media: Soybean casein broth, Columbia Agar
Differentiation media: Chromogenic Coliform Agar
Culture media: LB (similar growth also in enriched and soybean casein broths)
Bacterial growth: Growth abundant, large white-gray colonies, in liquid medium after inactivation easy to pellet by centrifugation
Biochemical features:

| **Name of the method:** ENTERO test 24N | | |
|---|---|---|
| **Identified strain:** *Escherichia coli* | | |
| **Evaluation:** Excellent identification (ID: 100, T-index: 0.924) | | |
| **Test** | **Abbreviation** | **Reaction** |
| Urease | URE | - |
| Arginine | ARG | - |
| Ornithine | ORN | + |
| Lysine | LYS | + |
| Hydrogen sulfide | H₂S | - |
| Simmons' citrate | SCI | - |
| Malonate | MAL | - |
| β-Galactosidase | ONP | + |
| Salicin | SAL | - |
| Sorbitol | SOR | + |
| Melibiose | MLB | + |
| Cellobiose | CEL | - |
| Lactose | LAC | + |
| Trehalose | TRE | + |
| Mannitol | MAN | + |
| β-Glucuronidase | GLR | + |
| Dulcitol | DUL | - |
| Adonitol | ADO | - |
| Arabitol | ART | - |
| Sucrose | SUC | + |
| Inositol | INO | - |
| Raffinose | RAF | + |
| Esculin | ESL | - |
| β-Xylosidase | bXY | - |

Antibiotic resistance:

| **Antibiotic abbreviation and concentration** | **Name of the antibiotic** |
|---|---|
| AMC 30 | Amoxicillin plus clavulanic acid |
| CL 30 | Cephalexin |
| CEP 30 | Cefoperazone |
| TE 30 | Tetracycline |
| N 30 | Neomycin |
| S 300 | Streptomycin |
| K 30 | Kanamycin |
| CN 10 | Gentamicin |
| MAR 5 | Marbocyl/Marbofloxacin |
| ENR 5 | Enrofloxacin |

| **Strain tested** | **Antibiotic** | | | | | | | | | |
|---|---|---|---|---|---|---|---|---|---|---|
| | **AMC 30** | **CL 30** | **CEP 30** | **TE 30** | **N 30** | **S 300** | **K 30** | **CN 10** | **MAR 5** | **ENR 5** |
| **PAN 4/1 *E. coli*** | MS | S | R | R | R | MS | R | MS | R | R |

| | | | | | | | | | | |
|---|---|---|---|---|---|---|---|---|---|---|
| S: sensitive MS: medium sensitive R: resistant | | | | | | | | | | |

### Genetic features:

The strain has the *elt* gene, encoding the LT heat-labile toxin in enterotoxigenic *E coli* (ETEC) strains.

### 5. Klebsiella oxytoca

### Immunolab Collection Number: 7L₂

Origin of the strain: Strain isolated from milk from a cow with mastitis symptoms. The sample was collected in January 2021 from the farm of Tadeusz Grzeń, designated 69149. Person collecting and transferring to the laboratory: vet. Przemys 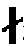 aw Warmowski.

Storage and transportation conditions: The milk sample was collected into a sterile container and frozen. After collecting more samples, they were transported under refrigeration to Immunolab company.
Growth media: Soybean casein broth, Columbia Agar with 5% sheep blood
Differentiation media: MacConkey Agar
Bacterial growth: Yellow, mucilaginous, surrounded by a zone of yellow colonies on MacConkey Agar, growth at 42°C, in liquid medium after inactivation difficult to pellet by centrifugation (wet, mucilaginous, spilling sediment)
Culture media: Enriched broth
Biochemical features:

| | | |
|---|---|---|
| **Name of the method:** ENTERO test 24N | | |
| **Identified strain:** *Klebsiella oxytoca* | | |
| **Evaluation:** Identification to genus (ID: 50.60, T-index: 0.971) | | |

| **Test** | **Abbreviation** | **Reaction** |
|---|---|---|
| Urease | URE | + |
| Arginine | ARG | - |
| Ornithine | ORN | - |
| Lysine | LYS | + |
| Hydrogen sulfide | H₂S | - |
| Simmons' citrate | SCI | + |
| Malonate | MAL | + |
| β-Galactosidase | ONP | + |
| Salicin | SAL | + |
| Sorbitol | SOR | + |
| Melibiose | MLB | + |
| Cellobiose | CEL | + |
| Lactose | LAC | + |
| Trehalose | TRE | + |
| Mannitol | MAN | + |
| β-Glucuronidase | GLR | - |
| Dulcitol | DUL | + |
| Adonitol | ADO | + |
| Arabitol | ART | + |
| Sucrose | SUC | + |
| Inositol | INO | + |
| Raffinose | RAF | + |
| Esculin | ESL | + |
| β-Xylosidase | bXY | + |

Antibiotic resistance:

| **Antibiotic abbreviation and concentration** | **Name of the antibiotic** |
|---|---|
| AMC 30 | Amoxicillin plus clavulanic acid |
| CL 30 | Cephalexin |
| CEP 30 | Cefoperazone |
| TE 30 | Tetracycline |
| N 30 | Neomycin |
| S 300 | Streptomycin |
| K 30 | Kanamycin |
| CN 10 | Gentamicin |
| MAR 5 | Marbocyl/Marbofloxacin |
| ENR 5 | Enrofloxacin |

| **Strain tested** | | **Antibiotic** | | | | | | | | | |
|---|---|---|---|---|---|---|---|---|---|---|---|
| | | **AMC 30** | **CL 30** | **CEP 30** | **TE 30** | **N 30** | **S 300** | **K 30** | **CN 10** | **MAR 5** | **ENR 5** |
| **7** | **L2** | S | S | MS | MS | MS | S | MS | MS | MS | MS |
| ***Klebsiella*** | | | | | | | | | | | |

| | | | | | | | | | | | |
|---|---|---|---|---|---|---|---|---|---|---|---|
| S: sensitive MS: medium sensitive R: resistant | | | | | | | | | | | |

### Genetic features:

The strain has the uge gene encoding the enzyme UDP-galactose-4'-epimerase, responsible for the synthesis of lipopolysaccharides, as well as the *kfu* gene responsible for the iron absorption system.

### 6. Klebsiella pneumoniae

### Immunolab Collection Number: 40 MO₂

Origin of the strain: The strain was isolated from milk from a cow with mastitis symptoms. The sample was collected in May 2021 from the Staśczat B. farm in the village of Kosiny Bartosowe, Wiśniewo municipality. Received from PAS Olsztyn.

Storage and transportation conditions: The milk sample was collected into a sterile container and frozen. After collecting more samples, they were transported under refrigeration to Immunolab company.
Growth media: Soybean casein broth, Columbia Agar with 5% sheep blood
Differentiation media: Chromogenic Coliform Agar
Bacterial growth: Mucilaginous blue-violet colonies on Chromogenic Coliform Agar, growth at 37 °C, in liquid medium after inactivation difficult to pellet by centrifugation (wet, mucilaginous, spilling pellet)
Culture media: Enriched broth
Biochemical features:

| | | |
|---|---|---|
| **Name of the method:** ENTERO test 24N | | |
| **Identified strain:** *Klebsiella pneumoniae subsp. pneumoniae* | | |
| **Evaluation:** Identification to genus (ID: 73.30, T-index: 1.000) | | |

| **Test** | **Abbreviation** | **Reaction** |
|---|---|---|
| Urease | URE | + |
| Arginine | ARG | - |
| Ornithine | ORN | - |
| Lysine | LYS | + |
| Hydrogen sulfide | H₂S | - |
| Simmons' citrate | SCI | + |
| Malonate | MAL | + |
| β-Galactosidase | ONP | + |
| Salicin | SAL | + |
| Sorbitol | SOR | + |
| Melibiose | MLB | + |
| Cellobiose | CEL | + |
| Lactose | LAC | + |
| Trehalose | TRE | + |
| Mannitol | MAN | + |
| β-Glucuronidase | GLR | - |
| Dulcitol | DUL | - |
| Adonitol | ADO | + |
| Arabitol | ART | + |
| Sucrose | SUC | + |
| Inositol | INO | + |
| Raffinose | RAF | + |
| Esculin | ESL | + |
| β-Xylosidase | bXY | + |

Antibiotic resistance:

| **Antibiotic abbreviation and concentration** | | | | | | | **Name of the antibiotic** | | | | |
|---|---|---|---|---|---|---|---|---|---|---|---|
| AMC 30 | | | | | | | Amoxicillin plus clavulanic acid | | | | |
| CL 30 | | | | | | | Cephalexin | | | | |
| CEP 30 | | | | | | | Cefoperazone | | | | |
| TE 30 | | | | | | | Tetracycline | | | | |
| N 30 | | | | | | | Neomycin | | | | |
| S 300 | | | | | | | Streptomycin | | | | |
| K 30 | | | | | | | Kanamycin | | | | |
| CN 10 | | | | | | | Gentamicin | | | | |
| MAR 5 | | | | | | | Marbocyl/Marbofloxacin | | | | |
| ENR 5 | | | | | | | Enrofloxacin | | | | |

| **Strain tested** | | **Antibiotic** | | | | | | | | | |
|---|---|---|---|---|---|---|---|---|---|---|---|
| | | **AMC 30** | **CL 30** | **CEP 30** | **TE 30** | **N 30** | **S 300** | **K 30** | **CN 10** | **MAR 5** | **ENR 5** |
| **40** | **MO₂** | W | W | MS | W | MS | W | O | MS | MS | MS |
| ***Klebsiella*** | | | | | | | | | | | |

| | | | | | | | | | | | |
|---|---|---|---|---|---|---|---|---|---|---|---|
| S: sensitive MS: medium sensitive R: resistant | | | | | | | | | | | |

### Genetic features:

Serotype K1, has a gene encoding aerobactin. It is a citrate-hydroxamate siderophore produced by many pathogenic Gram-negative bacteria to promote iron assimilation.

### 7. Staphylococcus aureus

### Immunolab Collection Number: 4.2 LG₂

Origin of the strain: A strain isolated from milk from a cow with mastitis symptoms. The sample was collected in March 2021 from the farm of Mariusz Kijańczyk in the village of G 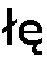 bock.

Storage and transportation conditions: The milk sample was collected into a sterile container and transported under refrigeration to Immunolab company.
Growth media: Soybean casein broth, Columbia Agar with 5% sheep blood
Differentiation media: Columbia CNA Agar, Chromogenic S. aureus Agar
Bacterial growth: Yellow, shiny, beta-hemolytic colonies on Columbia CNA medium, growth at 37 °C; pink colonies on Chromogenic S. aureus medium, growth at 37 °C, after culturing in liquid medium after inactivation easy to pellet by centrifugation, pellet strongly yellow or orange, growth very abundant
Culture media: Enriched or soybean casein broths
Biochemical features:

| | | |
|---|---|---|
| **Name of the method:** STAPHYtest 24 | | |
| **Identified strain:** *Staphylococcus aureus subsp. aureus* | | |
| **Evaluation:** Good identification (ID: 93.30, T-index: 0.618) | | |

| **Test** | **Abbreviation** | **Reaction** |
|---|---|---|
| Urease | URE | + |
| Arginine | ARG | + |
| Ornithine | ORN | - |
| β-Galactosidase | bGA | - |
| β-Glucuronidase | GLR | - |
| β-Glucosidase | bGL | + |
| Phosphatase | PHS | + |
| Esculin | ESL | - |
| N-acetyl-β-D-glucosamine | NAG | + |
| Galactose | GAL | + |
| Sucrose | SUC | + |
| Trehalose | TRE | + |
| Mannitol | MAN | + |
| Maltose | MLT | + |
| Xylose | XYL | - |
| Mannose | MNS | + |
| Lactose | LAC | - |
| Sorbitol | SOR | - |
| Ribose | RIB | - (visual evaluation) |
| Fructose | FRU | + |
| Cellobiose | CEL | - |
| Arabinose | ARA | - (visual evaluation) |
| Raffinose | RAF | - |
| Xylitol | XOL | - |

Catalase test: positive (gas bubble formation)
Serological features:
The strain was tested with the Staph Latex Kit (Pro-Lab) latex test: positive (aggregate formation)
Antibiotic resistance:

| **Antibiotic abbreviation and concentration** | **Name of the antibiotic** |
|---|---|
| AMC 30 | Amoxicillin plus clavulanic acid |
| P 1 | Penicillin |
| CX 5 | Cloxacillin |
| CL 30 | Cephalexin |
| CEP 30 | Cefoperazone |
| CFQ 30 | Cefquinome |
| TE 30 | Tetracycline |
| N 30 | Neomycin |
| S 300 | Streptomycin |
| K 30 | Kanamycin |
| CN 10 | Gentamicin |
| B 10 | Bacitracin |

| **Strain tested** | | **Antibiotic** | | | | | | | | | | | |
|---|---|---|---|---|---|---|---|---|---|---|---|---|---|
| | | **AMC 30** | **P 1** | **CX 5** | **CL 30** | **CEP 30** | **CFQ 30** | **TE 30** | **N 30** | **S 300** | **K 30** | **CN 10** | **B 10** |
| **4.2** | **LG₂** | S | S | MS | S | MS | MS | MS | MS | S | MS | MS | S |
| ***S*. *aureus*** | | | | | | | | | | | | | |

| | | | | | | | | | | | | | |
|---|---|---|---|---|---|---|---|---|---|---|---|---|---|
| S: sensitive MS: medium sensitive R: resistant | | | | | | | | | | | | | |

### Genetic features:

The strain expresses a wide range of virulence factors that include surface proteins covalently attached to the cell wall and proteins secreted during infection. These proteins include: **IsdC** - iron-regulated surface determinant protein C, **EsxA** - ESAT-6 secretory system extracellular protein, **IsdA** - surface adhesin, involved in iron sequestration, **IsdB** - adhesin, **Bap** - surface protein involved in biofilm formation, **SdrD, SrdC** - a subfamily of cell wall-anchored proteins called MSCRAMMs (microbial surface component recognizing adhesive matrix molecules) mediate adhesion to host tissues.

### 8. Streptococcus dysgalactiae

### Immunolab Collection Number: 2MO₂

Origin of the strain: Strain isolated from milk from a cow with mastitis symptoms. The sample was collected in March 2021, designated No. 2. Received from PAS Olsztyn.

Storage and transportation conditions: The milk sample was collected into a sterile container and frozen. After collecting more samples, they were transported under refrigeration to Immunolab company.
Growth media: Soybean casein broth, Columbia Agar with 5% sheep blood
Differentiation media: Columbia CNA Agar, Chromogenic Strepto B Agar
Bacterial growth: White-gray fine beta-hemolytic colonies on Columbia CNA Agar, growth at 37 °C; blue colonies on Chromogenic Strepto B Agar, growth at 37 °C, growth not very abundant in liquid medium, white pellet after centrifugation, inactivated antigen has a lumpy structure; no tube agglutination test possible
Culture media: Enriched broth or soybean casein broth (when cultured with shaking in flasks use EB, biofilm forms in TSB, when cultured in bioreactor biofilm does not form)
Biochemical features:

| **Name of the method:** STREPTOtest 16 | | |
|---|---|---|
| **Identified strain:** *Streptococcus dysgalactiae subsp. equisimilis* | | |
| **Evaluation:** Identification to species (ID: 65.20, T-index: 1.000) | | |
| **Test** | **Abbreviation** | **Reaction** |
| Hippurate | HIP | - |
| Phosphatase | PHS | + |
| Leucine aminopeptidase | LAP | + |
| B-Glucuronidase | GLR | + |
| A-Galactosidase | aGA | - |
| Esculin | ESL | - |
| Arginine | ARG | + |
| Urease | URE | - |
| Mannitol | MAN | - |
| Sorbitol | SOR | - |
| Trehalose | TRE | + |
| Lactose | LAC | + |
| Raffinose | RAF | - |
| Inulin | INU | - |
| Mellibiose | MLB | - |
| Ribose | RIB | + |

Catalase test: negative result (no gas bubbles)
Serological features:
The strain was tested with the Streptococcal Grouping Latex Kit (Pro-Lab) for typing streptococci into groups A, B, C, D, F, G according to Lancefield: positive with latex for group C (agglutination).
Antibiotic resistance:

| **Antibiotic abbreviation and concentration** | **Name of the antibiotic** |
|---|---|
| AX 25 | Amoxicillin |
| P 1 | Penicillin |
| AM 10 | Ampicillin |
| CX 5 | Cloxacillin |
| CL 30 | Cephalexin |
| CEP 30 | Cefoperazone |
| CFQ 30 | Cefquinome |
| E 15 | Erythromycin |
| TE 30 | Tetracycline |
| N 30 | Neomycin |
| L 15 | Lincomycin |
| B 10 | Bacitracin |

| **Strain tested** | | **Antibiotic** | | | | | | | | | | | |
|---|---|---|---|---|---|---|---|---|---|---|---|---|---|
| | | **AX 25** | **P 1** | **AM 10** | **CX 5** | **CL 30** | **CEP 30** | **CFQ 30** | **E 15** | **TE 30** | **N 30** | **L 15** | **B 10** |
| **2** | **MO2 S. dysgalactiae** | R | R | R | MS | S | R | R | R | R | MS | MS | MS |

| | | | | | | | | | | | | | |
|---|---|---|---|---|---|---|---|---|---|---|---|---|---|
| S: sensitive MS: medium sensitive R: resistant | | | | | | | | | | | | | |

### Genetic features:

The strain has the *eno* gene, encoding α-enolase. It is a glycolytic enzyme that is found on the surface of most streptococcal species as a multifunctional protein. It is involved in pathogenicity through plasminogen binding and cell adhesion/invasion. The strain also has the napr gene, encoding a plasminogen-binding receptor associated with nephritis.

### 9. Streptococcus uberis

### Immunolab Collection Number: 7MO

Origin of the strain: Strain isolated from milk from a cow with mastitis symptoms. The sample was collected in Match 2021 from the farm of Marek Wroński in the village of Szczepkowo-Giewarty, Janowo municipality, designated 4/2021 MO, 143 PT. Received from PAS Olsztyn.

Storage and transportation conditions: The milk sample was collected into a sterile container and frozen. After collecting more samples, they were transported under refrigeration to Immunolab company.
Growth media: Soybean casein broth, Columbia Agar with 5% sheep blood
Differentiation media: Columbia CNA Agar, Chromogenic Strepto B Agar
Bacterial growth: Blue-gray small colonies on Columbia CNA Agar, growth at 37 °C; blue colonies on Chromogenic Strepto B Agar, growth at 37 °C, growth not very abundant in liquid medium, white pellet after centrifugation
Culture media: Enriched or soybean casein broths
Biochemical features:

| **Name of the method:** STREPTOtest 16 | | |
|---|---|---|
| **Identified strain:** *Streptococcus uberis* | | |
| **Evaluation:** Very good identification (ID: 98.60, T-index: 1.000) | | |
| **Test** | **Abbreviation** | **Reaction** |
| Hippurate | HIP | + |
| Phosphatase | PHS | - |
| Leucine aminopeptidase | LAP | + |
| β-Glucuronidase | GLR | + |
| α-Galactosidase | aGA | - |
| Esculin | ESL | + |
| Arginine | ARG | + |
| Urease | URE | - |
| Mannitol | MAN | + |
| Sorbitol | SOR | + |
| Trehalose | TRE | + |
| Lactose | LAC | + |
| Raffinose | RAF | - |
| Inulin | INU | - |
| Mellibiose | MLB | - |
| Ribose | RIB | + |

Catalase test: negative result (no gas bubbles)
Serological features:
The strain was tested with the Streptococcal Grouping Latex Kit (Pro-Lab) for typing streptococci into groups A, B, C, D, F, G according to Lancefield: negative for all latexes (no agglutination).
Antibiotic resistance:

| **Antibiotic abbreviation and concentration** | **Name of the antibiotic** |
|---|---|
| AX 25 | Amoxicillin |
| P 1 | Penicillin |
| AM 10 | Ampicillin |
| CX 5 | Cloxacillin |
| CL 30 | Cephalexin |
| CEP 30 | Cefoperazone |
| CFQ 30 | Cefquinome |
| E 15 | Erythromycin |
| TE 30 | Tetracycline |
| N 30 | Neomycin |
| L 15 | Lincomycin |
| B 10 | Bacitracin |

| **Strain tested** | **Antibiotic** | | | | | | | | | | | |
|---|---|---|---|---|---|---|---|---|---|---|---|---|
| | **AX 25** | **P 1** | **AM 10** | **CX 5** | **CL 30** | **CEP 30** | **CFQ 30** | **E 15** | **TE 30** | **N 30** | **L 15** | **B 10** |
| **7MO S. *uberis*** | O | S | O | O | S | O | O | MS | O | O | O | S |

| | | | | | | | | | | | | |
|---|---|---|---|---|---|---|---|---|---|---|---|---|
| S: sensitive MS: medium sensitive R: resistant | | | | | | | | | | | | |

### Genetic features:

The strain has genes: *sua* - gene encoding adhesin protein, responsible for adhesion and invasion of epithelial cells, *pauA*/*skc* - plasminogen activators, responsible for colonization, *gapC* - encodes surface dehydrogenase protein, responsible for colonization, *hasA, hasB, hasC* - hyaluronic acid capsules, responsible for resistance to phagocytosis, *oppF* - gene encoding an oligopeptide permease co-forming oligopeptide transport system.

### 10. Streptococcus uberis

### Immunolab Collection Number: 19MK

Origin of the strain: Strain isolated from milk from a cow with mastitis symptoms. The sample was collected in February 2021 from a farm near Kartury. The sample was collected and donated by vet. Przemys 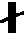 aw Warmowski.

Storage and transportation conditions: The milk sample was collected into a sterile container and transported under refrigeration to Immunolab company. The sample was stored in a freezer.
Growth media: Soybean casein broth, Columbia Agar with 5% sheep blood
Differentiation media: Columbia CNA Agar, Chromogenic Strepto B Agar
Bacterial growth: Blue-gray small colonies on Columbia CNA Agar, growth at 37 °C; blue colonies on Chromogenic Strepto B Agar, growth at 37 °C, growth not very abundant in liquid medium, white pellet after centrifugation
Culture media: Soybean casein or enriched broths
Biochemical features:

| **Name of the method:** STREPTOtest 16 | | |
|---|---|---|
| **Identified strain:** *Streptococcus uberis* | | |
| **Evaluation:** Very good identification (ID: 98.60, T-index: 1.000) | | |
| **Test** | **Abbreviation** | **Reaction** |
| Hippurate | HIP | + |
| Phosphatase | PHS | - |
| Leucine aminopeptidase | LAP | + |
| β-Glucuronidase | GLR | + |
| α-Galactosidase | aGA | - |
| Esculin | ESL | + |
| Arginine | ARG | + |
| Urease | URE | - |
| Mannitol | MAN | + |
| Sorbitol | SOR | + |
| Trehalose | TRE | + |
| Lactose | LAC | + |
| Raffinose | RAF | - |
| Inulin | INU | - |
| Mellibiose | MLB | - |
| Ribose | RIB | + |

Catalase test: negative result (no gas bubbles)
Serological features:
The strain was tested with Streptococcal Grouping Latex Kit (Pro-Lab) for typing streptococci into groups A, B, C, D, F, G according to Lancefield: negative result for all latexes (no agglutination).
Antibiotic resistance:

| **Antibiotic abbreviation and concentration** | **Name of the antibiotic** |
|---|---|
| AX 25 | Amoxicillin |
| P 1 | Penicillin |
| AM 10 | Ampicillin |
| CX 5 | Cloxacillin |
| CL 30 | Cephalexin |
| CEP 30 | Cefoperazone |
| CFQ 30 | Cefquinome |
| E 15 | Erythromycin |
| TE 30 | Tetracycline |
| N 30 | Neomycin |
| L 15 | Lincomycin |
| B 10 | Bacitracin |

| **Strain tested** | **Antibiotic** | | | | | | | | | | | |
|---|---|---|---|---|---|---|---|---|---|---|---|---|
| | **AX 25** | **P 1** | **AM 10** | **CX 5** | **CL 30** | **CEP 30** | **CFQ 30** | **E 15** | **TE 30** | **N 30** | **L 15** | **B 10** |
| **19MK *S*. *uberis*** | R | R | R | R | R | R | R | R | R | R | R | R |

| | | | | | | | | | | | | |
|---|---|---|---|---|---|---|---|---|---|---|---|---|
| S: sensitive MS: medium sensitive R: resistant | | | | | | | | | | | | |

### Genetic features:

The strain has the *cfu* gene, which encodes the cAMP factor, responsible for creating pores in the host cell membrane, allowing entry into the cell, and the *gapC* gene, which encodes the surface dehydrogenase protein, responsible for colonization.

### PRZYKtADY

### Example 1

### Inhibition of pathogen growth by IgY isolated from chicken egg yolks

The objective of the experiments is to test the ability of IgY antibodies to inhibit and/or limit bacterial growth. The study was conducted for all strains selected for hen immunization to produce antibodies. In the study, the starting amount of bacteria in the medium was 10⁴ CFU/mL; in milk cultures, such results are most often obtained for the total amount of bacteria. Antibody was used in two different amounts: 0.1 mg/10⁴ CFU and 0.05 mg/10⁴ CFU (IgY concentration measured for the total amount of IgY and not specific in the antibody series produced).

The treatment scheme for each strain was the same, the media used and the antibody series differed.

A preliminary test was performed to select appropriate dilutions for culture at the proper test. An inoculum of 10⁴ CFU/mL was used at 0, 3, 6 and 24 hours.

A culture was run in LB broth to rejuvenate the cultures of *E. coli* 16L1, PAN4/1, 4MO1 and 13MO2 strains and cultured overnight at 37 °C with shaking.

Flasks were prepared with 20 mL of medium, 3 for each strain - 1: control, 2: 0.1 mg, 3: 0.05 mg.

The turbidity of overnight cultures at a dilution of 1/100 was measured and CFU/mL was calculated.

The equivalent of 2×10⁵ CFU was added to each flask yielding 10⁴ CFU/mL and then IgY was added (list of series used see the results table).

Culture was run at time 0 from each control flask on EasySpiral without dilution (seeding 50 µl, circle mode, 1 replicate).

After 6h of culture, serial dilutions were prepared and cultures were run from each flask. 10⁻¹ was seeded for IgY cultures and for control cultures-a dilution of 10⁻⁵, circle mode, 50 µL, 2 replicates.

After 12h of culture, serial dilutions and cultures were made from each flask. 10⁻² was seeded for IgY culture and for the control cultures-a dilution of 10⁻⁶, circle mode, 50 µL, 2 replicates.

A second dose of IgY was added at 12h *(the product is to be administered to cows for 6 days every 12h, mimicking target conditions).*

Culture results from 0, 6 and 12h times were read, CFU/mL was calculated for each reading and averages were calculated.

After 24h of culture, serial dilutions and cultures were made from each flask. 10⁻² was seeded for IgY cultures and for control cultures-a dilution of 10⁻⁶, circle mode, 50 µL, 2 replicates. The culture results from the 24h time were read. CFU/mL was calculated for each reading and averages were calculated.

The test was repeated analogously for Klebsiella 40 MO₂ and 7 L2 strains and *S. aureus* 4.2 LG₂ strain (on TSA medium). The test was repeated analogously for S. *uberis* 19MK and 7MO strains and *S. dysgalactiae* 2MO2 (on Columbia medium with blood).

### RESULTS

**Table 2. List of antibodies used:**

| | *E coli* | *Klebsiella* | *S. aureus* | *S. uberis* | *S. dysgalactiae* |
|---|---|---|---|---|---|
| Series used | MS/EC/026 | MS/KL/025 | MS/SA/035 | MS/SU/009 | MS/SD/007 |
| IgY concentration at 1x concentration (total) | 2.88 mg/mL | 2.72 mg/mL | 2.57 mg/mL | 3.17 mg/mL | 3.38 mg/mL |
| Volume used to obtain 100 µg/10⁴ CFU, (1x concentration). | 694 µL | 735 µL | 778 µL | 630 µL | 591 µL |
| Volume used to obtain 50 µg/10⁴ CFU, (1x concentration). | 347 µL | 367 µL | 389 µL | 315 µL | 269 µL |

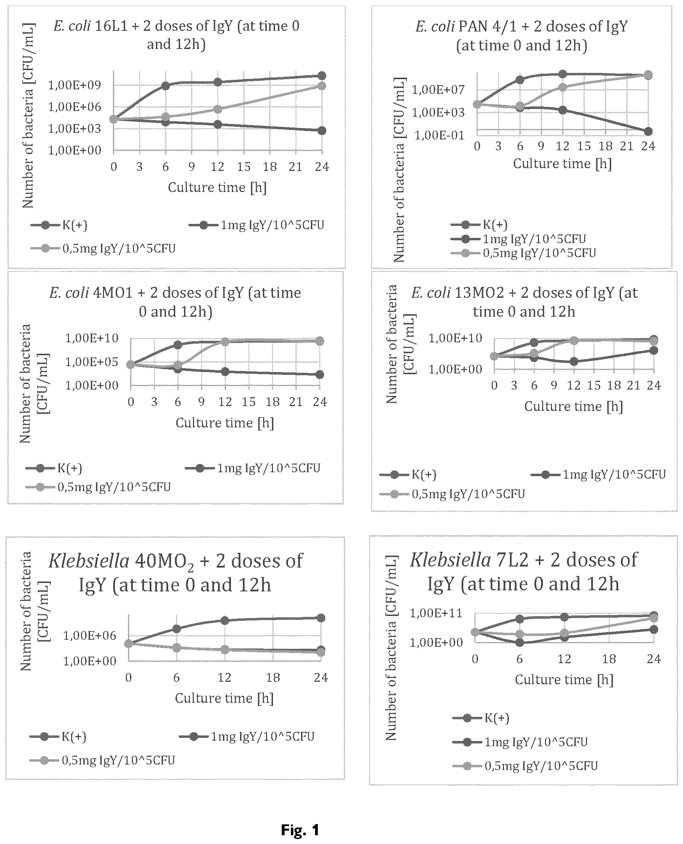

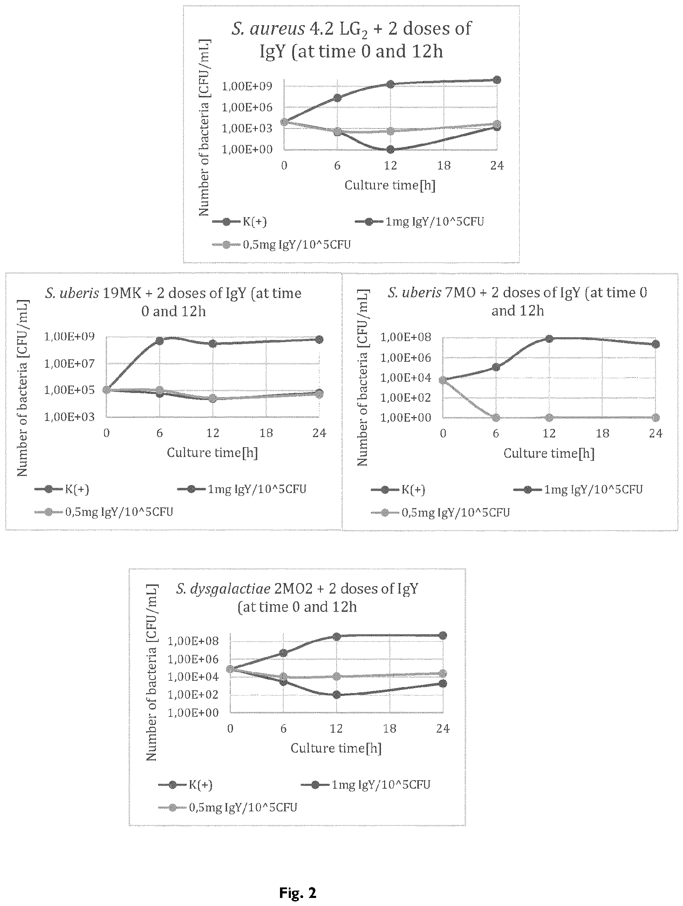

The results for *E coli* and Klebsiella are shown in Fig. 1, while the results for *S. aureus, S. uberis* and *S. dysgalactiae* are shown in Fig. 2.

### E. coli

For 16L1, PAN4/1 and 4MO1 strains, inhibition of culture growth was observed (40x, 530x and 130x smaller CFUs, respectively, after 24h than in the initial culture), after using two doses of antibodies 12h apart at 1 mg per 10⁵ CFU of the initial culture, indicating a strong bacteriostatic effect on these strains. Only in the case of 13MO2 strain the growth inhibition was less effective, but the bacteria did not grow as much as in the control culture (1000x fewer CFUs), indicating the inhibitory effect of IgY. Two doses of 0.5 mg per 10⁵ CFU of the initial culture did not show a growth inhibitory effect, to be measured after 6h of culture, the effect was evident, however, assuming doses every 12h, a higher dose (1 mg/10⁵ CFU) should be taken.

### Klebsiella

With the culture of 7L2 strain (*K*. *oxytoca*) the initial amount of bacteria was slightly lower than expected (instead of 10⁴ CFU/mL there were 8×10³ CFU/mL). The growth of 40 MO₂ strain (*K. pneumoniae*) was inhibited, in the case of *K. oxytoca* strain only partially for a dose of 1 mg/10⁵ CFU.

### S. aureus

When culturing *S. aureus,* the initial amount of bacteria was slightly lower than expected (instead of 10⁴ CFU/mL there were 8.5×10³ CFU/mL), therefore it should be assumed that it is better to use 1 mg per 10⁵ CFU of pathogen. Both doses led to comparable growth inhibition (not total).

### S. uberis

In the case of 19MK strain, the initial culture was denser (instead of 10⁴ CFU/mL it was 10⁵ CFU/mL, but it was able to produce a bacteriostatic effect during the whole study period (the amount of bacteria dropped by half)). For 7MO strain, complete growth inhibition was observed.

### S. dysgalactiae

In the case of 2MO2 strain, there were also more bacteria in the initial culture (instead of 10⁴ CFU/mL there were 7.5×10⁴ CFU/mL), nevertheless a bacteriostatic effect was observed and the number of bacteria dropped 42-fold for the 1 mg dose.

### CONCLUSIONS

Studies indicate that IgY can effectively inhibit bacterial proliferation. **In the case of cultures after 12 and 24h for all IgY-treated strains, the colonies on the plates were very fine, barely grown, compared to the grown colonies of the control cultures. This indicates a great weakening of their viability due to the effect of IgY.** This result also indicates that each successive dose weakens the viability of the bacteria and after the assumed 12 doses, complete inhibition of growth will be possible.

Based on the obtained results, for adequate inhibition of bacteria in the therapeutic preparation, it is possible to use (the table assumes twice the dose used in the *in vitro* studies):

**Table 3.**

| | *E coli* | *Klebsiella* | *S. aureus* | *S. uberis* | *S. dysgalactiae* |
|---|---|---|---|---|---|
| Amount of IgY/mL of the preparation | 2 mg | 2 mg | 2 mg | 2 mg | 2 mg |
| Amount of IgY in a dose of 10 mL of the preparation | 20 mg | 20 mg | 20 mg | 20 mg | 20 mg |
| Amount of IgY for the entire treatment (12 doses) | 240 mg | 240 mg | 240 mg | 240 mg | 240 mg |
| Average total IgY concentration in isolates of 1x IgY | 2.48 mg/mL | 2.37 mg/mL | 2.12 mg/mL | 3.09 mg/mL | 2.92 mg/mL |
| Estimated volume of 4x IgY concentration per 10 mL dose | 2 mL | 2.1 mL | 2.3 mL | 1.6 mL | 1.7 mL |
| Estimated volume of 4x IgY concentration per therapy (12 doses) | 24 mL | 25.2 mL | 27.6 mL | 19.2 mL | 20.4 mL |

### Example 2

### Evaluation of the purity of IgY antibodies isolated from chicken egg yolks

The objective of the experiment was to evaluate the purity of IgY antibodies (series MS/SA/043, SU/045, SD/047; KL/049, SA/050, EC/065, KL/088, SA/094, SD/105, SD/109, SU/110, EC/111, SD/112, SA/113, SU/115) isolated from chicken egg yolks with water and activated carbon and precipitated with sodium chloride. Evaluation was done by vertical electrophoretic separation in polyacrylamide gel.

A 12.5% polyacrylamide gel was prepared and casted. The gel was stored overnight at 4 °C in a box filled with deionized water. Appropriate dilutions were prepared for each test series. The samples were diluted 40-fold, *i.e.* in a sterile way (at the burner), 2 µL of antibodies were taken from each series and added to 78 µL of 0.85% NaCl. Loading buffer (7 µL) was added to each sample. Standard antibodies were diluted 10x in 0.85% NaCl. The samples prepared in this way were heated for 10 minutes at 96 °C, and then centrifuged. The samples were applied in the order as marked on the gel; the mass marker in a volume of 4 µL was applied to the first well, the standard chicken IgY to the last well. Vertical electrophoretic separation was carried out first at 100V, then 160V. After the separation was completed, the gel was stained in Coomassie Blue by rocking with the dye on a lab cradle for 20 minutes. The gel thus stained was rinsed in dH₂O and then boiled in the microwave 4 times for 4 minutes each and analyzed.

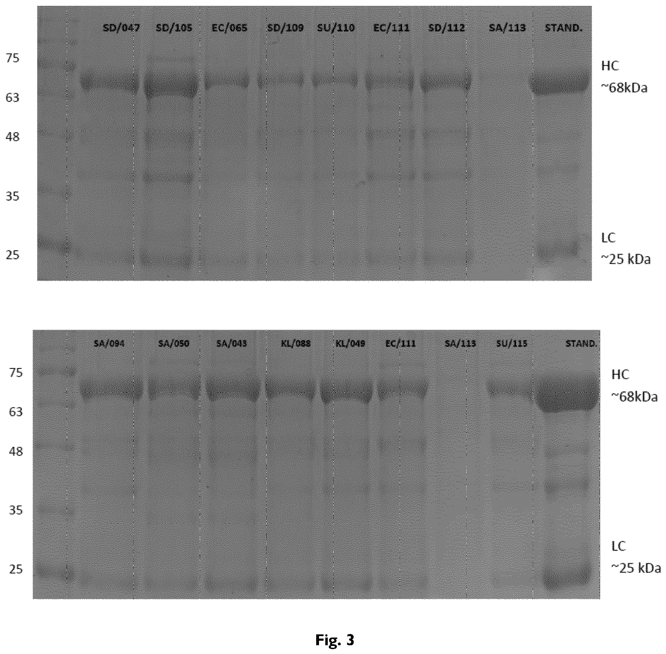

The tested series present a similar pattern during separation under denaturing conditions as standard chicken IgY (Fig. 3).

### CONCLUSIONS

Isolation of chicken egg yolks with water and activated charcoal and precipitation with sodium chloride yielded high quality and purity IgY antibodies.

### Example 3

### Study of the degree of E. coli bacteria neutralization with IgY antibodies encapsulated in hydrogel

The objective of the work performed was to determine the degree of neutralization of E. coli strains with IgY antibodies in a 0.5% hydrogel at a dose of 4 mg/mL.

Primary eukaryotic cells isolated from the milk-producing sinus of the cow's udder were seeded into 3 6-well plates and incubated in an incubator at 37 °C with 5% CO₂ until approximately 80% confluence was achieved. The cells were then infected with E coli bacteria (strains: 16L1, PAN4/1, 13MO2, 4MO1) at a dose of 10,000 CFU/mL, corresponding to the dose that was used to infect the cows. After 24 hours, the wells were washed 3 times with 1x concentrated PBS. DMEM with 1% bovine serum was added to 3 wells of each plate, and IgY antibodies in 0.5% hydrogel solution at a concentration of 4 mg/mL were added to the remaining 3 wells. The plates were incubated for 24, 48 and 72 hours, respectively, with rinsing and changing media every morning and after 8 hours. After the specified incubation time, cell lysates were prepared and seeded onto plates with CLA medium (Columbia) in several dilutions. The next day, bacterial colonies were counted using a Scan 500 device.

### Inoculation of overnight culture for infection

a. 30 mL of heated LB medium was inoculated with an *E. coli* culture consisting of the following strains: 16L1, PAN4/1, 13MO2, 4MO1.
b. The culture was incubated overnight at 37 °C.
c. The next day, the bacterial culture was rejuvenated in 30 mL of fresh LB medium at a ratio of 1:1000. After obtaining an OD₆₀₀ ~ 0.5, a dose was prepared for infection.

### Cell infection

a. Bacterial culture was re-suspended in serum-free medium (SFM) to achieve an optical density of 2 McF corresponding to 6×10⁸ CFU/mL.
b. From the initial dilution obtained, serial dilutions were prepared to obtain an amount of 10,000 CFU/mL in one well of the culture plate.
c. Eukaryotic cells were washed 3 times with 1 mL of SFM to flush away residual antibiotics included in the culture medium, incubating each time for 5 min in an incubator on a mixing platform.
d. An appropriate amount of bacterial suspension and SFM with 1% FBS were added to each well so that the final volume was 2 mL.
e. The infected cells were incubated for 24 hours.
f. After this time, the cells were washed 3 times with 1 mL of 1x concentrated PBS, incubating each time for 5 min in an incubator on a mixing platform.

### Testing the IgY preparation

a. 1 mL of SFM with 1% FBS (bacterial growth control) was added to 3 wells of each plate, and IgY antibody preparation in hydrogel at 4 mg/mL was added to the remaining 3 wells. Incubation was carried out for 24 hours in an incubator.
b. After this time, the culture wells were washed 3 times with 1 mL of 1x concentrated PBS, incubating each time for 5 minutes in the incubator on a mixing platform.
c. The following actions were then performed:
   1. For 24h time: 200 µL of lysis buffer was added to each well. After 20 min of lysis, the cells were scraped with a scraper and transferred to Eppendorf type tubes (3 wells each for the control and test samples were combined - a total of 600 µL for each sample).
   2. The samples were centrifuged for 10 min at 5000 rpm.
   3. For 48h and 72h times: After washing, 1 mL SFM with 1% FBS was added again to 3 wells of each plate, and antibody preparation was added to the remaining 3 wells.
   4. The media in the wells were changed after 8h of incubation and the next morning, each time rinsed with PBS (as in b) until the tests were completed for the given time.

### Preparation of lysates; test for bacteria neutralization degree

a. Lysates after centrifugation were transferred to new Eppendorf type tubes (supernatant).
b. Serial dilutions of lysates in sterile salt solution (10x⁻¹, ⁻² and ⁻³) were prepared.
c. Undiluted lysates, and the prepared serial dilutions were seeded onto CLA medium plates (two replicates for each sample) using easySpiral device (50 µL per plate).
d. Plates were read the next day using Scan 500 device.

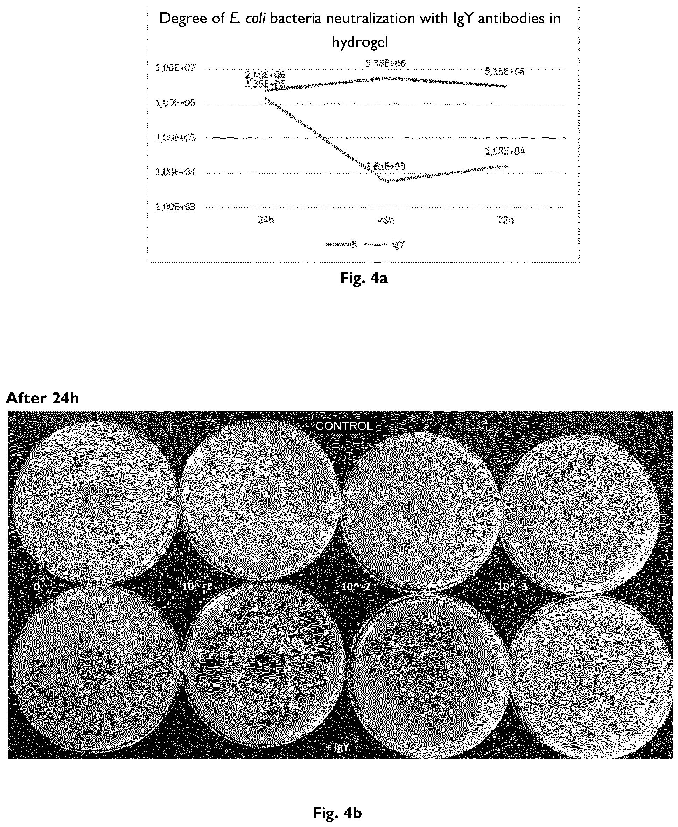

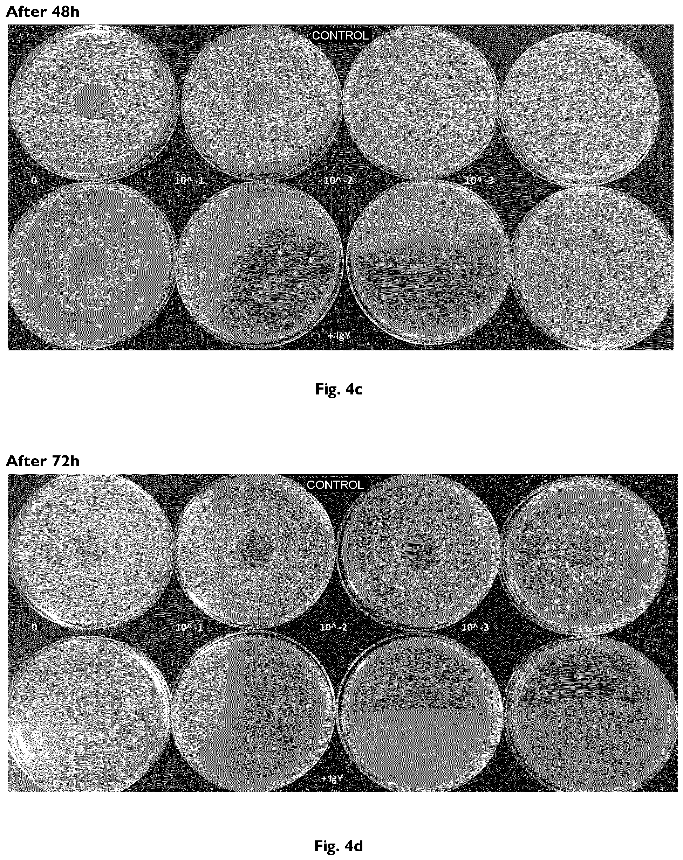

The neutralization results obtained are shown in Figs. 4a-4d.

### Example 4

*Study of the degree of S. dysgalactiae bacteria neutralization with IgY antibodies encapsulated in hydrogel*

The objective of the work performed was to determine the degree of neutralization of *S. dysgalactiae* bacteria with IgY antibodies in a 4 mg/mL hydrogel solution.

Primary eukaryotic cells isolated from the milk-producing sinus of the cow's udder were seeded into 3 6-well plates and incubated in an incubator at 37 °C with 5% CO₂ until approximately 80% confluence was achieved. The cells were then infected with *S. dysgalactiae* bacteria at 2500 CFU/mL, corresponding to the dose that was used to infect the cows. After 24 hours, the wells were washed 3 times with 1x concentrated PBS. DMEM with 1% bovine serum was added to 3 wells of each plate, and IgY antibodies in 0.5% hydrogel solution at a concentration of 4 mg/mL were added to the remaining 3 wells. The plates were incubated for 24, 48 and 72 hours, respectively, with rinsing and changing media every morning and after 8 hours. After the specified incubation time, cell lysates were prepared and seeded onto plates with CLA medium (Columbia) in several dilutions. The next day, bacterial colonies were counted using Scan 500 device.

### Inoculation of overnight culture for infection

a. 30 mL of heated LB medium was inoculated with a *S. dysgalactiae* culture.
b. The culture was incubated overnight at 37 °C.
c. The next day, the bacterial culture was rejuvenated in 30 mL of fresh LB medium at a ratio of 1:1000. After obtaining an OD₆₀₀ ~ 0.5, a dose was prepared for infection.

### Cell infection

a. Bacterial culture was re-suspended in serum-free medium (SFM) to achieve an optical density of 2 McF corresponding to 4×10⁸ CFU/mL.
b. From the initial dilution obtained, serial dilutions were prepared to obtain an amount of 2500 CFU/mL in one well of the culture plate.
c. Eukaryotic cells were washed 3 times with 1 mL of SFM to flush away residual antibiotics included in the culture medium, incubating each time for 5 min in an incubator on a mixing platform.
d. An appropriate amount of bacterial suspension and SFM with 1% FBS were added to each well so that the final volume was 2 mL.
e. The infected cells were incubated for 24 hours.
f. After this time, the cells were washed 3 times with 1 mL of 1x concentrated PBS, incubating each time for 5 min in an incubator on a mixing platform.

### Testing the IgY preparation

a. 1 mL of SFM with 1% FBS (bacterial growth control) was added to 3 wells of each plate, and IgY antibody preparation in hydrogel at 4 mg/mL was added to the remaining 3 wells. Incubation was carried out for 24 hours in an incubator.
b. After this time, the culture wells were washed 3 times with 1 mL of 1x concentrated PBS, incubating each time for 5 minutes in the incubator on a mixing platform.
c. The following actions were then performed:
   1. For 24h time: 200 µL of lysis buffer was added to each well. After 20 min of lysis, the cells were scraped with a scraper and transferred to Eppendorf type tubes (3 wells each for the control and test samples were combined - a total of 600 µL for each sample).
   2. The samples were centrifuged for 10 min at 5000 rpm.
   3. For 48h and 72h times: After washing, 1 mL SFM with 1% FBS was added again to 3 wells of each plate, and antibody preparation was added to the remaining 3 wells.
   4. The media in the wells were changed after 8h of incubation and the next morning, each time rinsed with PBS (as in b) until the tests were completed for the given time.

### Preparation of lysates; test for bacteria neutralization degree

a. Lysates after centrifugation were transferred to new Eppendorf type tubes (supernatant).
b. Serial dilutions of lysates in sterile salt solution (10x⁻¹, ⁻² and ⁻³) were prepared.
c. Undiluted lysates, and the prepared serial dilutions were seeded onto CLA medium plates (two replicates for each sample) using easySpiral device (50 µL per plate).
d. Plates were read the next day using Scan 500 device.

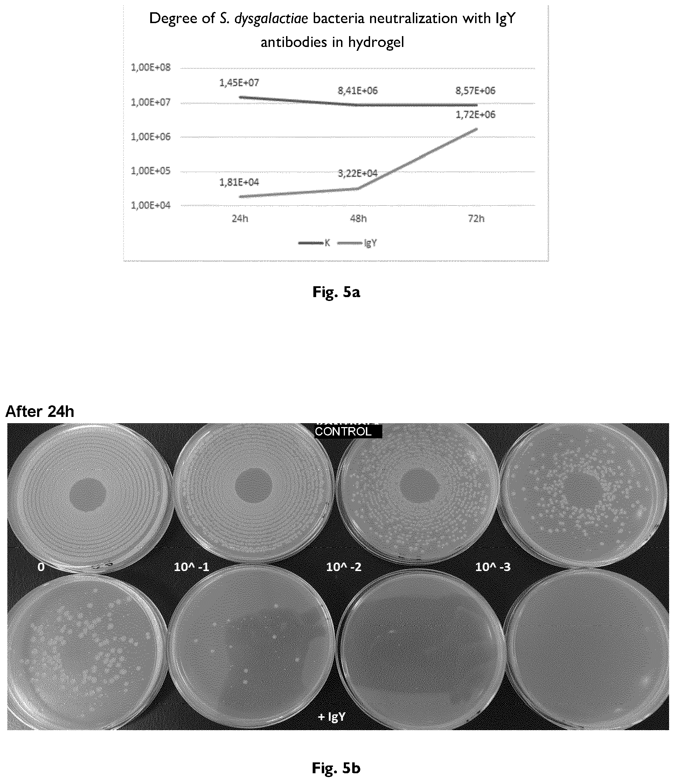

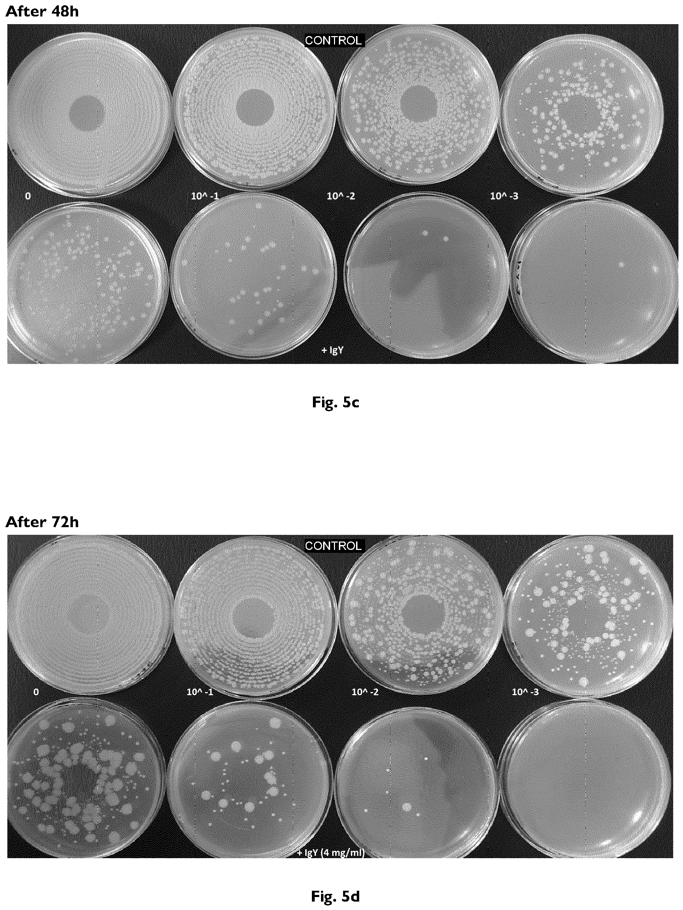

The neutralization results obtained are shown in Figs. 5a-5d.

### SUMMARY

### In vitro studies (example 1, 3 and 4)

In order to determine initial doses for testing on target animals, *in vitro* tests were conducted: a) Antibody neutralization of bacteria, testing the effect of different concentrations of IgY antibodies on pathogen growth on solid and liquid media in order to select the dose of antibodies for testing on cell lines; b) Testing the effect of IgY antibodies on bacterial proliferation in co-culture with primary cells isolated from the udder of a cow. An initial concentration was selected for the preparation of preparations for *in vivo* studies (4 mg/mL). Results for all pathogens tested showed a reduction in bacterial growth compared to the control by an average of 4 log.

### Example 5

### Preparation of a therapeutic preparation based on IgY antibodies in hydrogel and testing its efficacy

The objective of the work performed is to prepare a therapeutic preparation based on IgY antibodies in hydrogel. For the preparation of the drug, as before, IgY antibodies directed against 5 mastitis pathogens: *E coli, Klebsiella, S. aureus, S. uberis* and *S. dysgalactiae* were used. For the preparation of antibodies, antibodies selected taking into account the result of tube agglutination and the measurement of protein concentration (in the case of *S. dysgalactiae,* only the concentration) were used. It was assumed that the final protein concentration in the preparation should be 8 mg/mL (for each antigen). The drug would be administered 2x daily for 3 days.

Round V of the study involved testing 4 cows. Infection with *S. dysgalactiae* bacteria was conducted twice (morning and afternoon). Once symptoms of early mastitis were obtained, *i.e.* SCC min. 500,000, elevated body temperature, LDH, biochemistry and complete blood count, the medicinal product was administered in a volume of 5 mL to each infected teat. Hydrogel density: 0.5%.

### PREPARATION OF 0.5% HYDROGEL CONTAINING IgY ANTIBODIES

a. From each antigen a TOTAL I series was used, which is a 5-fold concentrated mixture of selected IgY antibody series.
b. A hydrogel containing 10% anhydrous glycerin and 1% hydroxyethylcellulose (HEC) with medium viscosity (1500 to 2500 cps) was prepared. 64.5 g of glycerin was weighed, taking into account its density (d=1.26 g/cm³), and 450 mL of water was added. 500 mL of solution was thus obtained, which was stirred and autoclaved. After sterilization, 5 g of hydroxyethylcellulose was poured into the hot solution and cooled on a magnetic stirrer until it reached room temperature.
c. An appropriate volume of 5x IgY was measured and filtered (0.22 µm) on a capsule filter under sterile conditions.
d. A total of 250 mL of filtered, 5x concentrated IgY and 281 mL of sterile saline according to the table were added to the 1% hydrogel thus prepared in a volume of 531 mL. The final concentration of IgY in the preparation is 8 mg/mL.
e. The finished hydrogel preparation was closed in 5 mL sterile tube syringes.

**Table 4.**

| Calculations for hydrogel 0.5% + IgY (16 mg/mL) | | | | | |
|---|---|---|---|---|---|
| | | **Abs** | **1x** | **5x** | **5x IgY in 100 mL** |
| **EC/total I** | *E*. *coli* | 0.657 | 4.94 | 24.70 | 64.8 |
| **SA/total I** | *S. aureus* | 0.658 | 4.95 | 24.75 | 64.6 |
| **SD/total I** | *S. dysgalactiae* | 0.560 | 4.21 | 21.05 | 76.0 |
| **KL/total I** | *Klebsiella* | 0.651 | 4.89 | 24.45 | 65.4 |
| **SU/total I** | S. *uberis* | 0.602 | 4.53 | 22.65 | 70.6 |

The sterility of the obtained preparation was confirmed.

### In vivo tests:

Four rounds of tests of the drug containing IgY antibodies were conducted *in vivo,* on cows. The safety and efficacy of the intramammary infusion with antibodies (therapeutic preparation) in the formulation, which was a hydrogel (L-H) drug, was determined.

Efficacy was determined in groups of cows infected with selected pathogens according to the scheme:
**(1) E. *coli* (n=18 cows), dosage** 4 mg/mL, 1x daily for 5 days
**(2) S. *uberis* (n=18 cows), dosage** (8 mg/mL) 2x daily for 3 days
**(3) S. *dysgalactiae* (n=18 cows), dosage** (8 mg/mL) 2x daily for 3 days
**(4) S. *aureus* (n=38 cows), dosage** (8 mg/mL) 2x daily for 3 days

In the group infected with a single pathogen, 3 subgroups were distinguished, consisting of:
- Infected cows untreated (infection control)
- Infected cows treated (treatment group)
- Uninfected cows treated (control)

Milk samples and blood from the external jugular vein were collected twice daily on days: -1, 0, 1, 2, 3, 4, 5, 7, 14, 21, 28, 35 and blood from the milk vein once daily. On day 35, udder tissue was collected. In the experiments, animal health and the following parameters were monitored:
**Milk analysis:** Although the drug administration inhibited inflammation, the high somatic cell count and LDH level persisted on average until day 21 and reached the initial level on day 35.

**Complete blood count:** Strong changes were observed in hematological blood parameters in cows with severe mastitis. An increase in neutrophils was predominant, and leukopenia in severe course.

**Blood biochemistry:** Health Check panel included: ALB, TP, GLOB, TB, AST, ALT, AMY, CK, CREA, BUN, GLU, TG, Ca, PHOS. Changes in parameters (mainly CK, CREA, TP) were observed in cows with severe induced mastitis, mainly *S. aureus.*

**ELISA:** Levels of TGF-β, PGE2 and PGF2α and mediators of ECM remodeling (MMP-9 and TIMP-1) changed during the course of the disease and treatment. A decrease in the amount of the studied factors was observed along with the healing process, the profile of which varied for the pathogens studied.

**Gene expression:** Pathogen-dependent changes in the expression of studied factors (Bax, BCL-2, CASP3, CASP8, RIPK1 and RIPK3, ELA, CAT, MPO, collagen I, III, IV, fibronectin, hyaluronic acid, MMP-9) were demonstrated. After treatment, degenerative and profibrotic processes were suppressed, and changes were observed mainly in genes related to tissue remodeling.

**Bacteria shedding (milk):** Log CFU below maximum value, SCC below normal range from day 14 or 21. The results correlate with antibody and individual cytokine levels. SCC in all study groups fell to the limits of the normal range around day 14-21 (in most cases the amount of bacteria remained below the acceptable limit during the whole period of the experiment).

**IgG (serum):** The level of IgG antibodies successively decreased from the beginning of the experiment in all experimental groups. Eventually, despite spikes in amounts, in each group the amount of antibodies on day 35 was lower than at the beginning of the study.

**IgM (serum):** During the course of the study, antibodies remained high in the group treated with liposomes with oleoamine, and a large spike was also observed on day 21 in the group treated with the oleoamine preparation. The rest of the groups, on the other hand, showed stable antibody levels during the whole period of experiment (all groups at similar levels).

**IgA (milk):** All test groups presented a similar pattern of IgA antibody levels; until day 7 of the experiment there was a very large decrease (to almost 0), followed by a significant increase. On the last day of the experiment, antibody levels dropped to the initial value or lower in all groups.

**TNF-α (serum):** Almost all groups showed an increase in the level of the factor from day 1 to day 3, followed by a sharp decrease. The group given empty liposomes with oleoamine showed an increasing trend in concentration throughout the study.

**TNF-α (milk):** The results of the TNF-α in milk obtained in the study confirmed the success of the infection carried out, which was particularly evident between infected and uninfected quarters. An increase in the factor could also be observed in the serum in the liposome-treated control group, indicating a strong ongoing inflammatory response in the body (compared to the group that was administered hydrogel with antibodies).

**IL-8 (serum):** In each group, the profile of serum interleukin levels differed. Elevated levels already at the beginning of the experiment were in the following groups: oleo liposomes-here, after a decrease on day 1, there was a significant increase and it persisted up to a week; in the groups which were given IgY in liposomes and empty hydrogel after a decrease on day 1, an increase was observed however, at the end of the experiment the amount of cytokine fell below the initial level (day 0). The situation was similar in the group which was given antibodies in hydrogel, however, in this group the level of interleukin was the lowest during the whole period of experiment.

**IL-8 (milk):** All **study groups** presented a very similar profile of interleukin levels. High increases were observed on days 1, 3 and 7, followed by a sharp decrease in interleukin in milk.

**IL-1β (serum):** All groups showed a low, similar pattern of interleukin levels during the whole period of experiment. The control group, which was given liposomes with oleoamine, presented a very high initial amount of IL-1β, after which a significant decrease was noted (1 day).

**IL-1β (milk):** The course of cytokine levels in all groups had a very similar profile during the whole period of the study. The highest increases were observed on days 2 and 4, followed by a sharp decrease in this factor.

The local immunologic response (milk) is significantly higher than that obtained from serum samples.

The drug was shown to be effective in inhibiting inflammations: 7 days after the end of drug administration, a return to initial parameters was observed, depending on the pathogen, in 30% to 70% of experimentally treated cows. Only higher level of SCC, as in standard antibiotic therapy, persisted in some cases until the day 21 after infection.

The original plan was: to use carriers in the form of liposomes with a cationic additive and 1% hydrogel, to use a dose of antibodies of 4 mg/mL in a treatment scheme of 2x daily for 6 days. Due to the results obtained (the irritant effect of the proposed carriers) and the welfare of animals in which the proposed dosing scheme could cause too much irritation, it was decided to make the following changes:
- dosage 2x daily for 3 days
- 8 mg/mL of IgY antibodies
- 0.5% hydrogel

The results obtained indicate that the use of the drug has a positive effect in case of mastitis. In most cases, a decrease in the somatic cell count (SCC) and the amount of bacteria in milk to the desired value, was achieved.

The efficacy of the adjunctive treatment of an intramammary infusion with a mixture of IgY was demonstrated. The preparation according to the present invention is effective, as the results of microbiological tests on the milk of the treatment groups indicate a lower number of CFU of mastitis-causing bacteria (at the level of significance p<0.05) compared to the control group.

Various versions of a local intramammary therapeutic preparation as an alternative to antibiotic therapy were designed, manufactured and tested both *in vitro* on cell lines and *in vivo* on target animals. A decrease in the somatic cell count (SCC) and a decrease in the number of bacteria in milk cultures in the following days after drug administration were obtained, demonstrating the therapeutic properties of the preparation.

Based on the results obtained, it should be concluded that the veterinary therapeutic preparation according to the present invention shows effective activity against mastitis. There are many factors that affect the efficacy of the drug, such as farming conditions or individual immune response of animals, seasonality, etc.

### Analysis of beneficial effects compared to other preparations:

Description of other preparations:
The competing products for the applicant's second product, i.e., an intramammary infusion containing a mixture of polyclonal IgY antibodies, are antibiotics. Among the antibiotic preparations used, the most commonly administered are those in the intramammary form:
Mastijet Forte/INTERVET/
Ubrostar Dcimm/BOEHRINGER/
Orbenin DC/ZOETIS/
Synulox LC/ZOETIS/
Cobactan LC/INTERVET/
Pathozone ZOETIS/

Such therapies have varied efficacy due to the rising of bacterial resistance to antibiotics, as well as untargeted treatment. Too frequent administration of antibiotics can cause allergies, skin lesions, exacerbate or over-inhibit cytokines, and eradicate the normal microflora.

Antibiotics, however, are not in direct competition with antibody intramammary infusion, but are an entity with partially the same functionality. The main advantages of the antibody infusion are the anticipated lack of withdrawal period for milk, the safety of use (no side effects) and the possibility of using it in organic farms due to its biological origin [Regulation (EU) 2018]. In addition, the antibodies present in the infusion do not induce bacterial resistance, which is a problem with antibiotics. There are currently no intramammary immunological therapeutic products for mastitis on the European market, which makes the product unrivaled.

The effect of IgY encapsulated in liposomes was tested for greater product efficacy. Although many studies indicate that liposomal forms of drugs have greater efficacy compared to standard forms [Sarecka-Hujar *et al.,* 2011] the present study did not show such an effect. Promising results were obtained for the liposomal preparation in terms of reducing bacterial shedding, although the addition of liposomes had an irritating effect on the teat. With animal welfare in mind, it was decided to continue the project by suspending the active ingredient in a hydrogel, which caused much less irritation to the teat. It is planned to refine the methods of liposome administration (optimization of liposome concentration, use of additives to minimize the risk of teat irritation, etc.). The use of liposomes may contribute to better penetration of antibodies into cells, deep into tissues or the biofilm formed by pathogenic bacteria, as well as result in longer persistence of antibodies in the body, potentially increasing the protective effect of the preparation, so it is planned to continue work on this variant of the therapeutic preparation.

### Brief description of drug preparation

Prepare the hydrogel (10% glycerol in water) and put it in an autoclave. After removing from the autoclave, put the hot hydrogel on a magnetic stirrer and after cooling to about 80-90 °C stirring all the time, add hydroxyethylcellulose powder, of medium viscosity (molecular weight of 170,000, viscosity of 1500-2500 cps), until a 0.5% concentration is obtained. Leave the hydrogel on the stirrer to cool completely. Filter the selected 5x concentrated mixture of selected IgY antibodies on a capsule filter (0.22 µm). Add concentrated IgY and sterile saline to the previously prepared hydrogel as calculated. The final concentration of IgY in the preparation is 8 mg/mL. Seal the prepared hydrogel preparation in sterile tube syringes in a volume of 5 mL.

### Other uses:

The proposed preparation (not an antibiotic) is an effective and innovative strategy for the treatment of udder inflammation in cows.

The drug, in the form of local therapy promotes alleviating bacterial udder inflammations of various etiologies and can find use as a complement to antibiotic therapy.

Effective treatment of udder inflammations in cows requires an individualized approach, taking into account a number of factors, such as the type of pathogen, the stage of the disease, the overall health of the animal and the specifics of the farming. If mastitis is suspected in cows, it is always advisable to consult a veterinarian, who can conduct the appropriate tests and plan the appropriate therapy.

## Claims

1. An immunizing and/or therapeutic preparation for use in the prevention and/or treatment of udder inflammation (mastitis) in cows, **characterized in that** it contains IgY antibodies isolated from chicken egg yolks, directed against:
a) *Escherichia coli*
b) *Klebsiella pneumoniae* and *Klebsiella oxytoca*
c) *Staphylococcus aureus*
d) *Streptococcus uberis*
e) *Streptococcus dysgalactiae*

2. The preparation according to claim 1, **characterized in that** the IgY antibodies are combined in a weight ratio of 1:1:1:1:1.

3. The preparation according to claim 1or 2, **characterized in that** the IgY antibodies are isolated from chicken egg yolks from hens immunized with the strain:
a) *Escherichia coli* having genes *stx1* and/or eae and *stx1* and/or *ipaH* and/or elt,
b) *Klebsiella pneumoniae* serotype K1, having the gene encoding aerobactin and Klebsiella *oxytoca* having the *uge* and *kfu* genes,
c) *Staphylococcus aureus* expressing the surface proteins IsdC, EsxA, IsdA, IsdB, Bap, SdrD, SrdC,
d) *Streptococcus uberis* having *sua, pauA*/*skc, gapC, hasA, hasB, hasC, oppF* and/or *cfu* and *gapC* genes,
e) *Streptococcus dysgalactiae* having eno and napr genes.

4. The preparation according to claim 1 or 2 or 3, **characterized in that** it is for intramammary administration.

5. The preparation according to any of claims 1-4, **characterized in that** it contains a carrier in the form of a hydrogel

6. The preparation according to claim 5, **characterized in that** the hydrogel contains 10% glycerol in water, 1% hydroxyethylcellulose powder with a viscosity of 1500 to 2500 cps.

7. The preparation according to any of the claims 1-6, **characterized in that** it contains saline and thimerosal 0.01% as additional substances.

8. The preparation according to any of the claims 1-7, **characterized in that** the final protein concentration in the preparation is 1 to 20 mg/mL for each antigen, preferably 2 to 10 mg/mL, more preferably 4 to 8 mg/mL.

9. The preparation according to any of the claims 1-8, **characterized in that** it is administered according to the following scheme:
| Administration | Age of the animal | Composition of the intramammary dose |
|---|---|---|
| 2x daily for 6 days | cows older than 24 months | 20 mg/mL of specific IgY antibodies (mixed in 1:1:1:1:1 weight ratio of 4 mg/mL of each antibody type) in a 1% hydrogel |
| 2x daily for 3 days | cows older than 24 months | 40 mg/mL of specific IgY antibodies (mixed in 1:1:1:1:1 weight ratio of 8 mg/mL of each antibody type) in a 0.5% hydrogel |

10. A method for obtaining a preparation as defined in any of claims 1-9, **characterized in that** it includes steps in which:
- a hydrogel containing 10% glycerol in water is prepared, heated to at least 95 °C, and then, after cooling to about 80-90 °C with stirring, hydroxyethylcellulose with a viscosity of 1500 to 2500 cps is added in powder form to obtain a concentration of 0.25 to 2%, preferably 0.5 to 1%, and the hydrogel is stirred until completely cooled;
- IgY mixture and sterile saline are added to the resulting hydrogel until the final IgY concentration in the preparation is 1 to 20 mg/mL for each antigen, preferably 2 to 10 mg/mL, more preferably 4 to 8 mg/mL.
